# EUROPEAN PATENT APPLICATION

(11) **EP 1 990 067 A2**
(43) Date of publication of application: **12.11.2008**
(21) Application number: 08161529.6
(22) Date of filing: 07.12.2006
(51) Int. Cl.: A61M 1/36

(54) **A pump-outflow-cannula and a blood managing system**

(30) Priority: 23.02.2006 US 776359 P; 22.05.2006 EP 06405219
(62) Divisional of application: 06125598.0
(71) Applicant: Levitronix LLC, Waltham, MA 02451 (US)
(72) Inventor: Gellman, Barry N., North Easton, MA 02356 (US); Richardson, J. Scott, Wilmington, MA 01887 (US); Marks, John, Salt Lake City, UT 84109 (US); Koert, Andrew, Somerville, MA 02144 (US)
(74) Representative: Sulzer Management AG

(57) **Abstract**

The invention relates to a pump-outflow-cannula (23) providing a blood conduit from an external blood handling system to a heart (2) and / or to an associated vessel, said pump-outflow-cannula (23) comprising a body (24) encompassing an essentially axially extending outflow-lumen (25) having a distal-end (26) for an introduction of blood from said blood management system into the outflow-lumen (25), and having a proximal-end (27) for an attachment of the outflow-lumen (25) to the heart (2) and / or to the associated vessel. According to the invention, said body (24) of the pump-outflow-cannula (23) comprises a reinforcement-means (28).The invention is also related to a blood managing system comprising a pump-outflow-cannula (23) in accordance with the present invention.

## Description

The invention relates to a pump-outflow-cannula providing a blood conduit from a heart and / or from an associated vessel, as well as to a blood managing system comprising a pump-outflow-cannula.

Cardiac support systems are utilized as a "bridge to decision" and a "bridge to transplant" for patients requiring physiologic cardiac stability. Such cardiac support systems are typically accomplished with an extracorporeal circuit containing a blood pump and sometimes an oxygenator which are attached to the blood circulation of a patient by a pump-inflow-cannula providing a blood conduit from the heart or from an associated vessel, e.g. from a vein to the blood pump and a pump-outflow-cannula providing a blood conduit from the blood pump back to the heart or an associated vessel, e.g. to an artery, in particular to the aorta. That is, the cardiac connection between the patient and the extracorporeal circuit is accomplished with said pump-inflow- and pump-outflow-cannulae that are placed within the cardiac chambers or major supply vessels.

Open heart surgery cannulae typically result in utilization of less than six hours while "bridge" cannulae or "long-term" cannulae may be used up to six month. Cannulae utilized for extended periods of time must impose minimal trauma on the blood.

For tong-term cardiac support up to six months or longer, the "UltraMag Blood Pump" from Levitronix LLC has turned out to work extremely reliable and producing minimal blood trauma. The same is true for Levitronix' "CentriMag Blood Pump" which is intended for short-term support, typically for less than 30-day use.

The most common techniques used in Cardiac Surgery Centers for postcardiotomy support include Extracorporeal Membrane Oxygenation (ECMO) and Ventricular Assist Devices (VAD) and Ventricular Assist Systems. Poor ventricular function may be diagnosed preoperatively or may have resulted from myocardial insult during surgery, for example from inadequate perfusion, cross-clamping for extended periods of time limiting reperfusion, injury, etc..

A reduced cardiac output over the years will affect other organs due to low blood pressure and blood flow. Over time, allowing the myocardium to rest may allow recovery. Thus, the patient may require long-term cardiac support. Patients who cannot be weaned from cardiopulmonary bypass and possess isolated ventricular dysfunction are probably candidates for a Ventricular Assist Device (VAD) or Ventricular Assist Systems (VAS). Also well known are BiVAD support systems requiring two-pump circuits. When pulmonary dysfunction occurs, the patient is most likely a candidate for Extracorporeal Membrane Oxygenation (ECMO).

Cardiac cannulae provide the patient interface means to an extracorporeal blood circuit. A placement of these cannulae may access the vasculature through major vessels, e.g. through Right Atrium (RA), Right Ventricle (RV), Left Atrium (LA), Left Ventricle (LV), allowing inclusion of trans-septal approach, Femoral Artery (FA), Femoral Vein (FV), Superior Vena Cava (SVC), Inferior Vena Cava (IVC) or the Aorta. Two cannulae are required in the extracorporeal circuit: one for pump inflow, the pump-inflow-cannula, and one for the pump outflow, the pump-outflow cannula.

The pump-inflow-cannula, sometimes referred to as the "venous cannula", is the primary conduit that transitions the blood from the patient to the extracorporeal circuit. The exact placement location is at the discretion of the surgeon. Ideally, the pump-inflow-cannula may be positioned within the ventricle transitioning the heart wall with the lumen of the cannula extending just past the wall. The cannula should be stabilized by a suture purse-string or a sewing ring to provide a means for securing the cannula against inadvertent dislodgement and to provide a leak free connection.

The pump-outflow-cannula, sometimes referred to as the "blood return cannula", or the "arterial cannula" (which may be a misnomer) is the primary conduit that transitions the blood from the extracorporeal circuit back to the patient. The aorta is the preferred site for the pump-outflow-cannula but other sites may be selected at the discretion of the physician. The pump outflow cannula may be secured through the aortic arch and may be accomplished by a variety of ways. One way is to secure a vascular graft to the transverse arch and pass the pump outflow cannula through the graft lumen, but preferably not enter into the vessel, and secure the graft to the cannula wrapping a suture about the graft. Another possibility may be to place the tip of the cannula through the wall of the aorta and stabilizing it with a purse-string suture or a tip stabilizing device. The pulmonary artery is also a common point of blood return.

The distal end of the pump-inflow-cannula, that is the end which is connected to the extracorporeal circuit, is passed through a dilated tunnel created from the ventricle through the subcutaneous plane to the percutaneous access site. The pump-outflow-cannula is passed through a dilated tunnel created from the arch of the ascending aorta through the subcutaneous plane to the percutaneous exit site. The percutaneous access sites are located ipsilaterally, on the left abdominal wall for the Left Ventricular Assist Device (LVAD), in the medial anterior position. The location is ipsilateral on the right abdominal wall for a Right Ventricular Assist Device (RVAD), in the medial anterior position. The extracorporeal system is attached to the pump-inflow-cannula and the pump-outflow-cannula using good perfusion technique. The open chest wound is closed upon successfully administrating the support system.

The support system, in particular the pump-inflow- and pump-outflow-cannulae known from the state of the art have several disadvantages, in particular with respect to the blood transfer from the heart and / or from the associated vessels into the pump-inflow-cannula as well as with respect to the transfer of the blood out of the pump-outflow-cannula into the heart or into the associated vessel.

The pump inflow cannula performs as a sump in the ventricle chamber. Thus, it is essential to inhibit the wall of the heart from being drawn into the low pressure orifice of the cannula lumen during blood aspiration. Such a condition can occlude the cannula, a problem which is not solved in a satisfactory manner up to now. Over more or less extended periods of time, very low flow rates can initiate blood clotting that can release and become lodges down-stream in the pump, oxygenator or patient organs. Furthermore, the attachment of the known cannulae to the patient is difficult to handle and, what is more, the known cannulae can be easily compressed and / or bended which can easily lead to a cross-clamping of the cannula, resulting in an interruption of the blood flow through the extracorporeal support system, which may cause serious consequences for the patient's physical health and, at worst, may lead to a life-threatening situation for the patient.

It is therefore an object of the invention to provide both, an improved pump-outflow-cannula as well as a blood managing system comprising such an improved cannula for establishing a blood conduit from a heart and / or an associated vessel to an external blood handling system, in particular for short-term applications. It is further an object of the invention to provide a method for connecting a cannula and a blood managing system in accordance with the invention to a heart and / or to an associated vessel of a human or an animal blood circulation and to propose a method for performing a bypass of a human or an animal organ.

The subject matter of the invention which satisfies these objects is characterized by the features of the independent claims of the respective category.

The respective subordinate claims relate to particularly advantageous embodiments of the invention.

The invention relates to a pump-outflow-cannula providing a blood conduit from an external blood handling system to a heart and / or to an associated vessel, said pump-outflow-cannula comprising a body encompassing an essentially axially extending outflow-lumen having a distal-end for an introduction of blood from said blood management system into the outflow-lumen, and having a proximal-end for an attachment of the outflow-lumen to the heart and / or to the associated vessel. According to the invention, said body of the pump-outflow-cannula comprises a reinforcement-means (28).

Before the pump-outflow-cannula of the present invention is discussed in greater detail, a pump-inflow-cannula is described which is not a subject matter of the present invention but can be advantageously combined with a pump-outflow-cannula of the present invention.

The aforementioned pump-inflow-cannula providing a blood conduit from a heart and / or from an associated vessel to an external blood handling system, said pump-inflow-cannula comprising a body, encompassing an inflow-lumen, extending essentially axially along a center-line between, having a distal-end for an attachment of the inflow-lumen to said blood handling system, and having a proximal-end for an introduction of blood from the heart and / or from the associated vessel into the inflow-lumen. At the proximal-end at least one angled-hole, extending around a longitudinal hole-axis is provided, and said hole-axis includes a presettable hole-angle with the center-line of the inflow-lumen, wherein said body of the pump-inflow-cannula comprises a reinforcement-means.

The pump-inflow-cannula comprises a reinforced body providing a blood conduit from the heart (or associated major vessel) extending transdermally to an externalized connection with the blood handling system. The cannula incorporates an essentially axial lumen, a proximal end for blood introduction and a distal end for attachment to the externalized blood handling system. As explained below, accessory components may affect an ease of device placement and setup for the user.

As already mentioned, the pump-inflow-cannula will perform as a sump, e.g. in the ventricle chamber. Therefore, it is essential to inhibit the wall of the heart from being drawn into the low pressure orifice of the pump-inflow-cannula lumen during blood aspiration. Such a condition would occlude the cannula. To minimize this condition, holes are placed peripherally about the walls of the pump-inflow-cannula at the tip so one or two lumen occlusion does not inhibit fully developed flow within the cannula. The placement of holes is commonplace for this function. In particular the proximal holes in the pump-inflow-cannula are angled relative to the centre-line of the cannula. This angling of the holes creates a slip-stream effect to eliminate (or greatly reduce) low flow areas within the cannula tip. Over extended periods of time, very low flow areas can initiate blood clotting that could release and become lodges downstream in the pump, oxygenator or patient Organs. The Computational Fluid Dynamics (CFD) analysis depicts this condition and could be verified in a real pump-inflow-cannula.

In a special embodiment, a pump-inflow-cannula comprises a reinforced body-portion to be essentially contained within a corpus, especially to be contained within the corpus and extending beyond a skin of the corpus, and comprises a compressible and / or bendable system-portion to be located outside of the corpus. The cannula portion contained within the body of the patient and slightly emerging is preferably wire reinforced to prevent collapse from tissue compression and / or from bending and must resist occlusion from low pressure created from a blood pump. The reinforcement-means of the pump-inflow-cannula emerging through the skin, should stop outside the emergence from the corpus of the patient to enable cross-clamping the cannula without cannula damage.

To maintain a small profile, the reinforcement-means are preferably located essentially within a wall structure of the body of the pump-inflow-cannula. In a special embodiment, the reinforcement means is a wire, in particular a round wire made of plastic and / or made of a composite material and / or made of a metal, especially made of a stainless steel and / or the reinforcement means is a spring, in particular a helical spring, maybe round or flattened spring and / or the reinforcement-means is encapsulated in a polymer.

Thus, the inflow-cannula provides both a blood supply to the extracorporeal blood-handling-system, e.g. to a blood pump, and structural support to the conduit.

In a special embodiment, a pump-inflow-cannula, includes at least two angled-holes, in particular two to six angled-holes, preferably six angled-holes, more preferably four angled-holes and a shape of a boundary of the angled-hole is circular and / or elliptical and / or tear drop shaped, and / or triangular, and / or trapezoid and / or elsewise shaped. Ideally, there should be at least wall thickness of material between holes about the cannula.

The boundary of the angled-hole is preferably circularly shaped having a diameter between 0.05" (0.127 cm) and 0.20" (0.508 cm), preferably between 0.10" (0.254 cm) and 0.15" (0.381 cm), more preferably 0.125" (0.3175 cm), in particular 0.13" (0.3302 cm).

The hole-angle included by the hole-axis and the centre-line of the inflow-lumen can be between 0° and 90°, preferably between 30° and 60°.

In an other embodiment which is very important in practice, an elongated-hole, in particular at least two elongated-holes, in more particular two to six elongated-holes, preferably six elongated-holes, more preferably an array of four elongated-holes are provided at the proximal-end, preferably at the proximal-end and distal to the angled-hole, and the elongated-hole is with respect to the centre-line preferably between 0.30" (0.762 cm) and 0.50" (1.27 cm) long, in particular 0.38" (0.9652 cm) long, and has a width between 0.05" (0.127 cm) and 0.20" (0.508 cm), preferably between 0.10" (0.254 cm) and 0.15" (0.381), more preferably 0.125" (0.3175 cm), in particular 0.13" (0.3302 cm), wherein, preferably, at a surface of the wall-structure of the angled hole and / or of the elongated-hole an edge-radius is provided to reduce trauma to the blood.

A surface of the pump-inflow-cannula is biocompatible to blood and / or a tissue and / or the pump-inflow cannula is made of a polycarbonate-based urethane, especially made of carbothane and / or another urethane und / or PVC, and / or vinyl materials, and / or elastomeric materials, and the wall-structure of the body of the pump-inflow-cannula, in particular the proximal-end of the pump-outflow-cannula, includes at least two layers, in particular including an inner soft-layer having a hardness between 40 Shore A and 120 Shore A, preferably between 50 Shore A and 100 Shore A, in particular 70 Shore A and / or including an outer hard-layer having a hardness between 40 Shore A and 120 Shore A, preferably between 50 Shore A and 100 Shore A, in particular 85 Shore A.

That is, the pump-inflow-cannula as well as the pump-outflow-cannula may be produced from polyvinyl chloride (PVC), polyurethane (PU), or other rubber-like elastic soft material and may be fabricated by a number of manufacturing processes. These include extrusion, and / or dip molding and / or injection molding or combinations there-of. The processing must preferably enable the inclusion of a spring / wire reinforcement-means within the wall of the cannula distal to the drainage holes. This reinforcement-means is, as mentioned, typically stainless steel but is not limited to that material. The material (cannula and support structure) must resist occlusion from low pressures created by the blood pump, purse-string sutures applied by the user to secure the cannula within the body, suturing about the cannula to create hemostasis, maintenance of luminal patency during cannula bending and oscillation within the body, forces applied from tissues and muscle groups in contact with from tunneling.

To further improve the pump-inflow-cannula and reduce trauma to the blood, the holes punched through the cannula wall should contain radii at the wall surfaces. It is important to reduce all sharp edges in the cannula as sharp edges cause blood trauma. The pump-inflow-cannula, which is the patient outflow cannula, is thus preferably constructed of a number of layered material durometers. The proximal cannula tip may consist in particular of two material durometers. Material that creates the inside diameter of the proximal tip is in a preferred embodiment a softer material than that of the outer cannula surface. The inner cannula material consists of a softer material. The higher durometer material provides cannula stiffness while the other material will result in greater radii. It should be noted that more than two different material layers may be incorporated and the softer material layer may indeed be positioned outboard of the harder layer. Alternatively, three or more layers may be incorporated with two soft layers sandwiching a stiffer layer to result in large formed radii both inside and out.

The radii are added to hole edges by solvent dip (cyclohexanone, methylethylketone (MEK), or other solvent), heat and / or mechanical abrasion. The softer cannula material will result in a greater radii being formed. The inner wall intersection results in the greatest blood shear so a larger radii is preferred.

Regarding a preferred embodiment, for indicating a placement depth from the epicardial surface of the heart a depth-indicator may be provided and / or a radiopaque feature is provided comprising a reference feature to enable imaging of the proximal-end of the pump-inflow-cannula for post-surgical placement assessment and wherein the radiopaque feature is preferably a stainless steel marking-spring incorporated in the proximal end of the pump-inflow-cannula and / or the radiopaque feature may surround the inflow-lumen and / or the radiopaque feature is a marker made of tantalum and / or stainless steel and / or an other radiopaque material.

Depth marking indicators are important, because the pump-inflow-cannula is placed under direct vision. The pump-inflow-cannula must provide the physician an indication of the proximal tip location within the heart. Thus, the depth-indicator is provided on the pump-inflow-cannula body, indicating placement depth from the epicardial surface of the heart. These markings display 1-cm indicator lines from 3-cm to 10-cm on the pump-inflow-cannula, and from 1-cm to 10-cm on the pump-outflow-cannula, which is described below in more detail. The depth-indicators may be circumferential or sectors. Marking may or may not be radiopaque and / or may be imbedded in the cannula tip wall or be coated on the cannula wall surface.

The tip of the pump-inflow- cannula incorporates preferably a radiopaque feature to enable fluoroscopic imaging of the cannula tip for post-surgical placement assessment. This is accomplished for example by incorporating a stainless steel dot within 0.2" of tip. Alternatively, tantalum, 0.05" diameter or other radiopaque material marking feature may be utilized. The indicator provides a reference feature. Alternatively, the feature may surround the lumen (i.e.: cylinder) and may be imbedded in the cannula tip wall or be coated on the cannula wall surface. The size of the indicator should be a minimum of .025" projected area.

Regarding a special embodiment of a pump-inflow-cannula, a shaping-means, in particular a malleable wire is provided to shape the proximal-end of the pump-inflow-cannula. That is, the pump-inflow-cannula may additionally provide a means to permit the user to shape the proximal cannula end. Often when the cannula is placed through the atrium, the cannula tip is directed to the ventricle. To accomplish this, the cannula may be produced with a malleable wire preferably embedded in the cannula wall. The malleable wire is positioned on the outside of the reinforcing spring and anchored with mechanical undercuts to secure the proximal end. In this cannula, the distal malleable anchor is positioned within a tip area of harder polymer such to further reduce deflection and maintain stability of the joint. It is understood, that preferably the pump-inflow-cannula may comprise said shaping-means, but, in a special case, a pump-outflow-cannula according to the present invention may include such a shaping-means, too.

In a further embodiment, the pump-inflow-cannula and / or the pump-outflow cannula will include a positionable securing-grommet to secure the pump-inflow-cannula and / or the pump-outflow-cannula.

The cannula will contain one or more securing-grommets positionable along the cannula length for the surgeon to suture stain-relief at strategic locations along the implant. The securing-grommets should enable the cannula to be secured with sutures without resulting in the occlusion of the cannula lumen. The securing-grommets are designed to fit about the outside diameter of the cannula. Its shape should provide a means for wrapping a suture while providing a feature to keep the suture from inadvertently sliding off. The securing-grommet may be made from silicone, polyurethane, TPR rubber, or other elastomeric material compatible for long-term implant and / or may be injection molded, cast, or formed. Preferably, the securing-grommet may be placed on the cannula via the proximal end prior to surgery, or over the long axis of the cannulae post insertion.

The distal-end of a pump-inflow-cannula, includes preferably an inflow-fitting, in particular a barbed 3/8" (0.95 cm) fitting capable of receiving a 3/8" tube-connector, preferably a fitting comprising a countersink to receive a fitting-wall of the tube-connector, for attachment of the pump-inflow-cannula to the external blood handling system. The diameter may be smaller (1/4" diameter) for smaller patients and pediatric/neonates.

The barbed fitting to cannula joint have been designed to provide a smooth transition along the inner surface to minimize or eliminate any gap that could result in thrombosis. Thrombus will form at gaps or irregular surfaces and may grow and / or be accidentally released back into the blood stream. Such debris may become lodged downstream and could result in patient complications. To minimize this, the cannula body has preferably a countersink to receive the barbed fitting wall and result in no irregular surface transition. This feature is common also to the blood-outflow-cannula. A barbed fitting, if pre-attached to the cannula, should be protected during tissue tunnelling or passage from damage and contamination. Thus, a tunneler cap component, also called tunneler-plug, may be added to the device as described below.

The present invention is also related to a pump-outflow-cannula providing a blood conduit from an external blood handling system to a heart and / or to an associated vessel. Said pump-outflow-cannula comprises a body encompassing an essentially axially extending outflow-lumen having a distal-end for an introduction of blood from said blood handling system into the outflow-lumen, and having a proximal-end for an attachment of the outflow-lumen to the heart and / or to the associated vessel, wherein said body of the pump-outflow-cannula comprises a reinforcement-means.

Regarding a preferred embodiment of a pump-outflow-cannula in accordance with the present invention, the pump-outflow-cannula comprises a reinforced body-portion to be essentially contained within a corpus of a human or an animal, especially to be contained within the corpus and extending beyond a skin of the corpus, and comprising a compressible and / or bendable system-portion to be located outside of the corpus.

The reinforcement-means are preferably located within a wall structure of the body of the pump-outflow-cannula and / or the reinforcement-means may be a wire, in particular a wire made of plastic, and / or made of a composite material, and / or made of a metal, especially made of a stainless steel. The reinforcement-means can be a spring , in particular a helical spring, especially a round or flattened spring and in more particular, the reinforcement-means is encapsulated in a polymer.

Thus, the pump-outflow-cannula in accordance with the present invention provides both a blood supply from the extracorporeal blood-handling-system, e.g. from a blood pump, and structural support to the conduit.

The pump-outflow-cannula may be produced from polyvinyl chloride (PVC), polyurethane (PU), or other rubber-like elastic soft material and may be fabricated by a number of manufacturing processes. These include extrusion, dip molding and / or injection molding or combinations there-of. The processing must enable the inclusion of a reinforcement-means, e.g. a wire and / or a spring within the wall of the cannula distal to the drainage holes. This reinforcement is typically stainless steel but is not limited to that material. The material (cannula and support structure) must resist occlusion from low pressures created by the blood pump, purse-string sutures applied by the user to secure the pump-outflow-cannula within the body of a patient, suturing about the cannula to create hemostasis, maintenance of luminal patency during cannula bending and oscillation within the body, forces applied from tissues and muscle groups in contact with from tunneling.

A pump-outflow-cannula in accordance with the invention includes at the distal-end of the outflow-lumen an outflow-fitting, in particular a barbed 3/8" fitting capable of receiving a 3/8" tube-connector, preferably a fitting comprising a countersink to receive a fitting-wall of the tube-connector, for attachment of the pump-outflow-cannula to the external blood handling system and / or wherein the distal-end is straight or angled according to an distal-angle, the distal-angle preferably ranging from 0° to 40° from a outflow-lumen-axis, preferably ranging from 1° to 30°, in particular being 10°. The diameter can be smaller (1/4" diameter) for smaller patients and pediatric / neonates.

The interface between the barbed fitting and the pump-outflow-cannula should provide a smooth transition along the blood flow lumen resulting in essentially no voids to hold blood or protrusions to impose undue shear on the blood. As such, the cannula design includes a relief in the material wall to align the connector inside diameter with the cannula inside lumen. As already mentioned, the distal cannula tip may be straight or angled slightly.

In a special embodiment, the proximal-end of the pump-outflow-cannula comprises a securing-means for securing the proximal-end to the heart and / or to the associated vessel, in particular a vascular graft.

The proximal-end of the outflow-lumen is preferably a through lumen comprising at a hardened proximal-tip, and the periphery about the proximal-tip ranges from 0.005" (0.127 mm) to 0.800" (20 mm), in particular from 0.100" (2.54 mm) to 0.600" (15.24 mm), and is preferably 0.315" (8 mm).

The proximal end of the blood return cannula may be placed within the lumen of a vascular graft (8-mm graft), preferably attached to the aortic artery. The graft is intended to prevent accidental decannulation and to reduce pressure necrosis of the vessel wall by providing a "soft" interface, and a smooth cannula to vessel transition. Placement of the cannula within the graft and not extending the cannula into the aorta reduces dislodge of embolic material from the return blood flow stream. The proximal lumen of the blood return cannula is a through lumen. The material tip should be hardened to resist deformation. This may be accomplished by solvent dipping to extract plasticizer (if PVC or vinyl) or locally dipped into a harder material durometer.

In a special embodiment, the proximal-end of a pump-outflow-cannula preferably comprises a fluid-deflection for controlling the blood flow into a vessel, in particular a fluid-deflection with hardened deflection-tip, wherein the deflection-tip is straight or bend 1° to 60°, preferably 5° to 30° from an longitudinal axis of the outflow-lumen.

Regarding a special embodiment which is very important in practice, a surface of the pump-outflow-cannula is biocompatible to blood and / or a tissue and / or the pump-outflow-cannula is made of a polycarbonate-based urethane, especially made of carbothane and / or another urethane und / or PVC, and / or vinyl materials, and / or elastomeric materials, and / or the cannula material durometer ranges from 50 Shore A to 100 Shore A, preferably from 70 Shore A to 90 Shore A and / or the pump-inflow-cannula is fabricated by dip-molding, extrusion, injection molding or combinations thereof and / or is fabricated in a single polymer layer and / or in multiple polymer layers and / or in sections hermetically bonded.

Preferably, a depth-indicator is provided for indicating a placement depth into the vessel and / or a positionable securing-grommet is provided to secure the pump-outflow-cannula.

The pump-outflow-cannula as well as the pump-inflow-cannula is typically placed in the patient under direct vision. The cannula must provide the physician an indication of the proximal tip location within the heart and / or within a vessel.

Thus, depth-indicators may be provided on the cannula body indicating placement depth into the aorta or into the vascular graft conduit. These markings display preferably 1-cm indicator lines (range from 1-cm to 10-cm) from the most distal opening. Depth markings may be circumferential or sectors. Marking may or may not be radiopaque. These depth markers may be imbedded in the cannula tip wall or be coated on the cannula wall surface.

In addition, the pump-outflow-cannula will include a number of securing-grommets positionable along the cannula length for the surgeon to suture stain-relief at strategic locations along the implant. The securing-grommets should be sized to the appropriate cannula outside dimensions (not necessarily the same sizes as the pump-inflow-cannula). These grommets should function the same as the pump-inflow-cannula grommets. The number of grommets utilized is at the discretion of the physician to hold the internal cannula location and shape. The securing-grommet may be placed on the cannula via the proximal end prior to surgery, or over the long axis of the cannulae post insertion.

Preferably, an introducer with a hub including a guide wire is provided, in particular a guide wire having a diameter of 0.038", to permit access into a vessel, in particular into the aorta.

The guide wire is slideably fit into the central lumen of the introducer and is used to exact cannula placement by means of providing a directed insertion path along the body of the introducer into the blood vessel according to the Seldinger (or other) technique. The introducer provides a resistance to arterial blood backflow up the cannula. The slow removal of the introducer provides a controlled reversed priming of the cannula before the user clamps the cannula readying it for assembly to the circuit. The introducer is seated within a seal affixed over the barbed fitting on the distal cannula end. This seal provides a wiper for the introducer to prevent blood flow leakage as the introducer is withdrawn from the cannula. A barbed fitting, if pre-attached to the cannula, should be protected during tissue tunnelling or passage from damage and contamination. A tunneler-plug component may be added to the device. This tunneler-plug may be the same as the pump-inflow-cannula tunneler-plug.

The invention is furthermore concerned with a blood managing system comprising a pump-inflow-cannula and / or a pump-outflow-cannula in accordance with the present invention.

A blood managing system in accordance with the invention, comprises preferably a tunneler-plug to assist the pump-inflow-cannula and / or the pump-outflow-cannula in tunneling through a tissue of a corpus. To assist in tunneling the cannula through the tissue, the tunnel-plug is preferably attached to the distal-end into the exit lumen of the respective cannula. The tunneler-plug can include a barbed fitting on one end and a smooth tapering section on the other end to ease passage as it (with its assembled cannula) dilates a lumen during passage.

The tunneler-plug may be manually attached and removed from the cannula fitting. A double start luer-like thread attachment means may be employed but sized to a dimension greater than the introducer seal contained within. The end of the tunneler-plug may contain an eyelet to which a suture or tape may be attached to provide a grasping feature to aid in pulling the device through the tissue. Alternatively, an extension rod or a screw-like or locking coupling to attach an extension rod may be incorporated to assist in tissue passage. The tunneler-plug profile must provide a means to dilate protect the components within the cap from debris to maintain sterile connector. Also the profile must provide a smooth transition to easily permit tunnel passage of the tunneler-plug followed by coupled cannula.

The tunneler-plug is made of rigid polymer or metal (polycarbonate is preferred) and may be produced by injection molding, machining, casting or stereolithography (rapid prototyping) for short term body contact. The distal tunneler-plug may also include a means to secure a suture or umbilical tape to create a tether for assisting in pulling the assembly through the tissue.

Upon completion of passage of the cannula outside the body, the tunneler-plug may be disconnected or cut-off from the cannula and the distal portion of the cannula is attached to the extracorporeal blood handling system.

Prior to insertion of the cannula into the ventricle of the heart, to securely locate the pump-inflow-cannula into a heart, a sewing-ring is provided.

The sewing-ring provides a means to securely locate in particular the blood-inflow-cannula into the heart. The sewing-ring is attached to the apex of the Left Ventricle by means of direct vision with the aid of a temporary positioning handle. The sewing-ring is configured intended to provide a mechanical means to secure the cannula to the heart by providing radial compression to the cannula while mechanically fastened to the heart tissue with sutures. The sewing-ring consists of a cylindrical body sized to receive the cannula outside diameter with minimal clearance.

The construction of the sewing-ring includes silicone material and polyester felt. Alternatively, any hemocompatible thermoplastic or thermoset elastomer may be utilized. The design is essentially a "top-hat" configuration with the top removed. The brim provides the location for securing sutures to the heart as well as position of the felt disc for tissue interface. Included in this flange is a reinforcement member that is intended to minimize suture pull-through (bolster) as the felt material may not provide the needed structural resistance. The upper "top-hat" portion includes pre-assembled umbilical tape. This enables less manipulation by the user to satisfactorily tie-off the cannula. The umbilical tape provides a larger surface area while wrapping to resist locally cutting through the sewing-ring body and cannula wall.

Prior to implantation of the pump-inflow-cannula and / or of the pump-outflow-cannula, the sewing-ring is preferably placed in proximity to the Left Ventricle and located such that the felt covered fabric side is facing the heart, and the central axis lumen is directly in-line with the apex of the left ventricle. The aid of the Handle provides a feature to manipulate the sewing ring during handling and attachment. Up to six or more pledged sutures placed through the heart tissue at even intervals approximately 60° circumferentially apart from each other around the periphery of the heart. The suture is brought through the heart tissue approximately 1-2 cm away from the apex of the heart and the final position of the sewing-ring. The sutures are pulled through the heart muscle leading away from the pledged and towards the ventricular apex. The sutures are brought through the tissue and externalized at the point that they enter the underside of the Sewing Ring. The ends of each unique pledgeted are string are brought through the cuff of the sewing-ring and securely fastened.

Once positioned securely on the heart, a stab wound or punch is utilized to create access to the inner chamber of the heart. The cannula is then passed through the sewing-ring into the heart. The cannula is positioned with the most distal drainage hole contained within the heart chamber. The umbilical tape that is pre-attached to the sewing-ring body is then wrapped about the cannula and secured with a knot to minimize cannula axial movement and blood leakage between the cannula and the sewing-ring cylinder.

In addition, the sewing-ring felt disc should enable clotting of any wicked blood under the sewing-ring to again inhibit blood leakage.

Regarding a further embodiment of a blood managing system in accordance with the present invention, a blood handling system, in particular a blood pump, or an oxygenator, especially an extracorporeal membrane oxygenator, or a dialysis apparatus, or an other medical apparatus is provided for connection with the pump-inflow-cannula and / or with the pump-outflow-cannula, in particular to connect the pump-inflow-cannula with the pump-outflow-cannula.

A method for connecting a pump-inflow-cannula to a heart and / or to an associated vessel of a human and / or an animal blood circulation comprises the following steps:
providing a pump-inflow-cannula for establishing a blood conduit from the heart and / or from an associated vessel to an external blood handling system, wherein said pump-inflow-cannula includes a body, encompassing an essentially axially extending inflow-lumen, having a distal-end for an attachment of the inflow-lumen to said blood management system, and having a proximal-end for an introduction of blood from the heart and / or from the associated vessel into the inflow-lumen, wherein at the proximal-end is at least one angled-hole provided, extending around a longitudinal hole-axis, and said hole-axis including a presettable hole-angle with the center-line of the inflow-lumen, wherein said body of the pump-inflow-cannula comprises a reinforcement-means;
fixing the proximal-end of the pump-inflow-cannula at the heart and / or at the associated vessel, in particular fixing the pump-inflow-cannula by a sewing technique.

A method for connecting a pump-outflow-cannula to a heart and / or to an associated vessel of a human and / or an animal blood circulation is comprising the following steps:
providing a pump-outflow-cannula for establishing a blood conduit from an external blood handling system to the heart and / or to an associated vessel, said pump-outflow-cannula comprising a body encompassing an essentially axially extending outflow-lumen having a distal-end for an introduction of blood from said blood management system into the outflow-lumen, and having a proximal-end for an attachment of the outflow-lumen to the heart and / or to the associated vessel, wherein said body of the pump-outflow-cannula comprises a reinforcement-means;
fixing the proximal-end of the pump-outflow-cannula at the heart and / or at the associated vessel, in particular fixing the pump-outflow-cannula by a sewing technique.

The invention is furthermore related to a method for connecting a blood managing system to a heart and / or to an associated vessel of a human and / or an animal blood circulation comprising the following steps:
connecting a pump-inflow-cannula to a heart and / or to an associated vessel and / or connecting a pump-outflow-cannula to a heart and / or to an associated vessel as described above;
connecting a pump-outflow-cannula to a heart and / or to an associated vessel and / or connecting a pump-inflow-cannula to a heart and / or to an associated vessel as described above.

Regarding a preferred method for connecting a blood managing system in accordance with the present invention, a blood handling system is provided, in particular a blood pump, or an oxygenator, especially an extracorporeal membrane oxygenator, or a dialysis apparatus, or an other medical apparatus and connecting said blood handling system with the distal-end of the pump-inflow-cannula and / or with the distal-end of the pump-outflow-cannula.

A method for performing a bypass of a human and / or of an animal organ, in particular for bypassing a heart, and / or for dialysis, and / or for a active suction or pump assisted autotransfusion, and / or for a cardio-pulmonary bypass surgery and or for performing a bypass within an other medical use, is comprising the following steps:
providing a pump-inflow-cannula for establishing a blood conduit from a heart and / or from an associated vessel to an external blood handling system, said pump-inflow-cannula comprising a body, encompassing an essentially axially extending inflow-lumen, having a distal-end for an attachment of the inflow-lumen to said blood management system, and having a proximal-end for an introduction of blood from the heart and / or from the associated vessel into the inflow-lumen, wherein at the proximal-end is at least one angled-hole provided, extending around a longitudinal hole-axis, and said hole-axis including a presettable hole-angle with the center-line of the inflow-lumen, wherein said body of the pump-inflow-cannula comprises a reinforcement-means;
and / or providing a pump-outflow-cannula for establishing a blood conduit from an external blood handling system to a heart and / or to an associated vessel, said pump-outflow-cannula comprising a body encompassing an essentially axially extending outflow-lumen having a distal-end for an introduction of blood from said blood management system into the outflow-lumen, and having a proximal-end for an attachment of the outflow-lumen to the heart and / or to the associated vessel, and said body of the pump-outflow-cannula comprises a reinforcement-means;
and / or providing a blood handling system, in particular a blood pump, or an oxygenator, especially an extracorporeal membrane oxygenator, or a dialysis apparatus, or an other medical apparatus;
and / or fixing the proximal-end of the pump-inflow-cannula at the heart and / or at the associated vessel, in particular fixing the pump-inflow-cannula by a sewing technique.
and / or fixing the proximal end of the pump-outflow-cannula at the heart and / or at the associated vessel, in particular fixing the pump-outflow-cannula is by a sewing technique;
and / or said blood handling system is connected with the distal-end of the pump-inflow-cannula and / or with the distal-end of the pump-outflow-cannula.

In the following, the invention will be explained in more detail with reference to the drawings. Shown are:
- Fig. 1:: a pump-inflow-cannula with angled-holes;
- Fig. 2:: a pump-inflow-cannula with elongated-holes;
- Fig. 3:: an angled-hole with edge-radii;
- Fig. 4:: a pump-outflow-cannula;
- Fig. 5:: a barbed fitting joint;
- Fig. 6:: a tunneler-plug;
- Fig. 6a: a tunneler-cap;
- Fig. 7:: a sewing-ring accessory;
- Fig. 7a:: a sewing-ring in accordance with Fig. 7 attached to a heart;
- Fig. 7b:: bottom view of Fig. 7a;
- Fig. 8:: a pump-outflow-cannula incorporating no vascular graft;

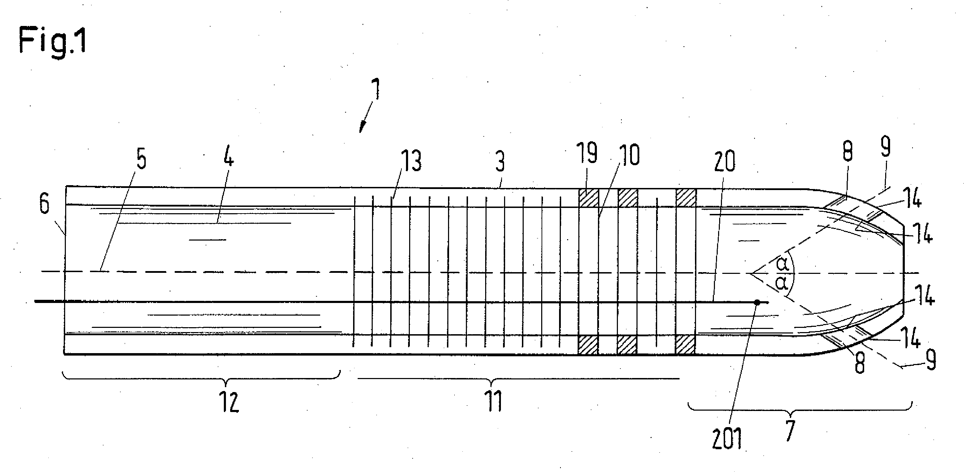

In Fig. 1 a pump-inflow-cannula with angled-holes is schematically displayed, which pump-inflow-cannula will be designated in the following by the reference number 1. The pump-inflow-cannula 1 providing a blood conduit from a heart 2 and / or from an associated vessel to an external blood handling system, is comprising a body 3, encompassing inflow-lumen 4, extending essentially axially along a center-line 5, having a distal-end 6 for an attachment of the inflow-lumen 4 to said blood handling system, and having a proximal-end 7 for an introduction of blood from the heart 2 and / or from the associated vessel into the inflow-lumen 4. In the present example of Fig. 1, the pump-inflow-cannula 1 comprises a blood flowing lumen, axial to the cannula, and includes four angled-holes 8 (only two angled-holes 8 can be seen in Fig. 1, because Fig. 1 is a sectional view of the pump-inflow-cannula 1) extending around a longitudinal hole-axis 9 and including a presettable hole-angle α with the center-line 5 of the outflow-lumen 4. The body 3 of the pump-inflow-cannula 1 comprises a reinforcement-means 10.

Since the pump-inflow-cannula 1 is placed under direct vision, it must provide the physician an indication of the proximal tip location, that is the location of the proximal-end 7 within the heart 2. Thus, depth-indicators 19 are provided at the body 3 of the pump-inflow-cannula 1 indicating placement depth from the epicardial surface of the heart 2. These depth-indicators 19 may display for example 1-cm indicator lines from 3-cm to 10-cm on the pump-inflow-cannula and from 1-cm to 10-cm on a pump-outflow-cannula. The depth-indicators 19 are circumferential or sectors and may or may not be radiopaque. In the present embodiment, the depth-indicators 19 are imbedded in the cannula wall, that is in the body 3 of the pump-inflow-cannula 1. In particular, the depth-indicators 19 may be imbedded in the cannula tip wall or be coated on the surface of the cannula wall-structure 31, 31.

In addition, at the pump-inflow-cannula 1 shown in Fig. 1 a shaping-means 20 to permit the user to axially shape the proximal-end 7 of the pump-inflow-cannula 1. Often when the pump-inflow-cannula 1 is placed through the atrium, the cannula tip is directed to the ventricle. To accomplish this, the pump-inflow-cannula 1 can be produced with a malleable-wire 20 embedded in the wall-structure 13. The malleable-wire 20 is positioned on the outside of the reinforcing spring 10 and anchored with mechanical means 201 such as undercuts 201, ribs 201 or holes 201 to secure the proximal-end 7. In the pump-inflow-cannula 1 of Fig. 1, the distal malleable anchor is positioned within a tip area of harder polymer such to further reduce deflection and maintain stability of the joint.

To provide biocompatible surfaces to the blood and tissue, the preferred cannula material is Carbothane (polycarbonate-based urethane) but other urethane, PVC, vinyl materials, or other elastomeric materials may be utilized.

The cannula body 3 is intended to provide a conduit from the blood supply to the extracorporeal device circuit located outside the body. Therefore, the body 3 of the pump-inflow-cannula 1 provides blood passage as well as structural support to the conduit. In addition, the cannula body 3 contacts both internal organs / tissues and external handling of the device.

The pump-inflow-cannula 1 is "tunneled" through a passage emerging out through the skin of a patient. The reinforced body-portion 11 of the pump-inflow-cannula 1 contained within the body 3 and slightly emerging is reinforced by a reinforcement-means 10 to prevent collapse from tissue compression and / or from bending and / or must resist occlusion from low pressure created for example by a blood-pump. In the example of Fig. 1 the reinforcement-means 10 is a wire 10 in form of a spring 10 made of stainless steel being totally encapsulated in a polymer and to maintain a small profile, the spring 10 is located within the wall-structure 13 of the body 3 of the pump-inflow-cannula 1.

The reinforcement should stop outside the emergence from the skin of the patient to enable cross-clamping the pump-inflow-cannula 1 without cannula damage. Therefore, the pump-inlow-cannula 1 of Fig. 1 includes a compressible and or bendable system-portion 12, allowing cross-clamping of the cannula to occlude the lumen.

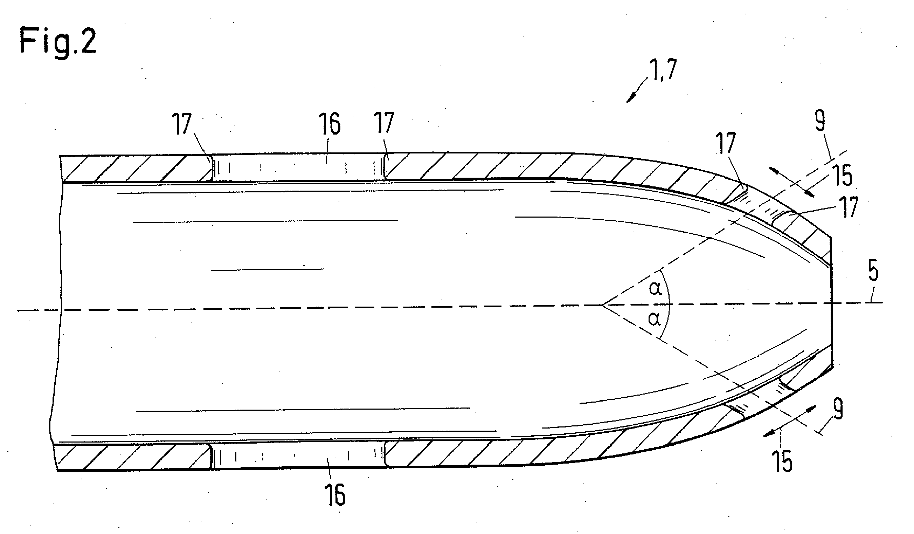

Fig. 2 shows in a sectional view a special embodiment of the proximal end 7 of a pump-inflow-cannula 1 having additionally elongated-holes. As already mentioned, the pump-inflow-cannula 1 performs as a sump in the ventricle chamber. And, thus, it is essential to inhibit the wall of the heart 2 from being drawn into the low pressure orifice of the inflow-lumen 4 during blood aspiration. Such a condition would occlude the pump-inflow-cannula 1.

To ovoid or minimize this condition, angled-holes 8 are placed peripherally about the proximal-end 7 at the tip of the pump-inflow-cannula 1, so that one or two lumen occlusion does not inhibit fully developed flow within the pump-inflow-cannula 1. The proximal angled-holes 8 in the pump-inflow-cannula 1 are angled relative to the center-line 5 of the pump-inflow-cannula 1. This angling of the angled-holes 8 creates a slip-stream effect to eliminate or greatly reduce low flow areas within the tip of the pump-inflow-cannula 1. Angled-holes 8 result in reducing flow dead-space within the flow area.

The hole-angle α of the angled-holes 8 range from 5° to 85° from the axial perpendicular, by more practically from 30° to 60°. The special embodiment shown in Fig. 2 incorporates round shaped angled-holes 8, but are not limited to such. The hole shape may also be elliptical, tear drop shape, triangular, trapezoid, etc..

In the present example of Fig. 2, there are 4 essentially round angled-holes 8 equally spaced about the cannula periphery (only two angled-holes 8 are shown in Fig. 2). The angled-holes 8 are 0.13" diameter 15 each, but it is understood, that the size of the angled-hole 8 can vary depending upon the number of holes desired and depending upon the diameter of the pump-inflow-cannula 1. The diameter 15 of the angled-hole 8 can range from 0.1" to 0.1 5", preferably a diameter 15 of 0.125".

Additionally, an array of four elongated-holes 16 (0.38" long X 0.13" diameter, only two are shown) are positioned distal to the first array of the angled-holes 8. The elongated-holes 16 are placed distal to the angled-holes 8 and are in the present example of Fig. 2 perpendicular to the axis once flow has been fully developed.

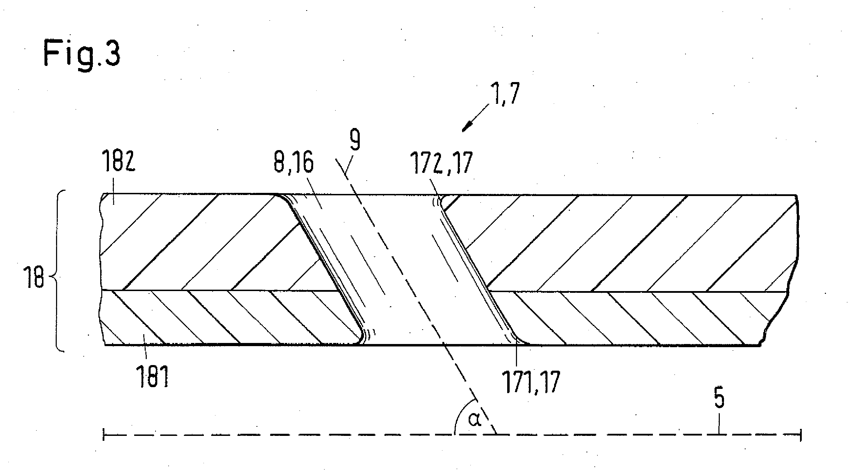

In Fig.3 a very important embodiment of a layer configuration of the proximal-end 7 of the pump-inflow-cannula 1 is displayed. The angled-hole 8 includes edge-radii 17 at the wall surfaces to reduce trauma to the blood. It is very important to reduce all sharp edges in the cannula as sharp edges cause blood trauma. The pump-inflow-cannula 1 is preferably constructed of a number of layered material durometers, that is, it can be constructed of two layers 18, for example an inner soft-layer 181 and an out hard-layer 182. The proximal cannula tip includes two material durometers. The Material that creates the inside diameter of the proximal tip is a softer material than that of the outer cannula surface.

The inner soft-layer 181 consists of a softer material. The hardness of this material can range from 50 Shore A to 100 Shore A, preferably 70 Shore A. The hardness of the outer hard-layer 182 can range from 50 Shore A to 100 Shore and is preferably 85 Shore A. The higher durometer inner soft-layer 181 provides the pump-inflow-cannula 1 stiffness, while the outer hard-layer 182 will result in greater radii 182. It should be noted that more than two different material layers 18 may be incorporated and the softer material layer may indeed be positioned outboard of the harder layer.

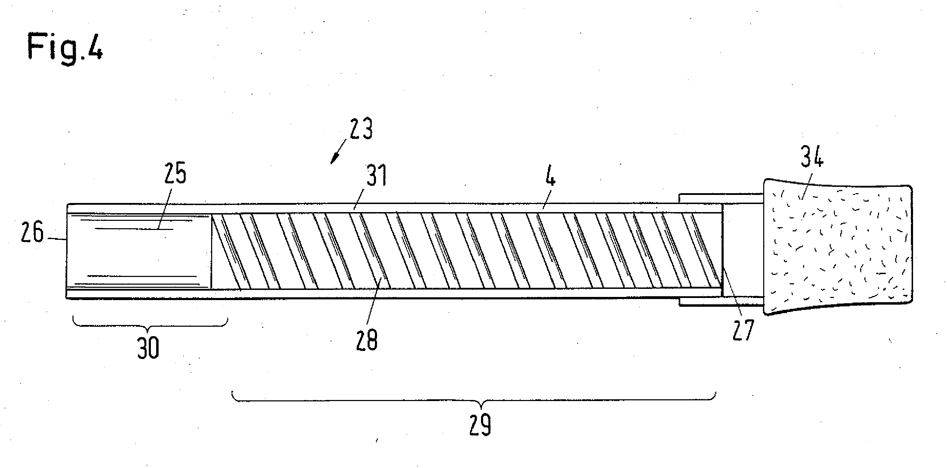

Fig. 4 shows a very simple embodiment of a pump-outflow-cannula 23 in accordance with the present invention. The pump-outflow-cannula 23 of Fig. 4, providing a blood conduit from an external blood handling system to a heart 2 and / or to an associated vessel, comprises a body 24 encompassing an essentially axially extending outflow-lumen 25, having a distal-end 26 for an introduction of blood from said blood management system into the outflow-lumen 23, and having a proximal-end 27 for an attachment of the outflow-lumen 25 to the heart 2 and / or to the associated vessel. According to the invention, said body 24 of the pump-outflow-cannula 23 comprises a reinforcement-means 28.

Thereby, the pump-outflow-cannula 23 displayed in Fig. 4, comprises a reinforced body-portion 29 to be essentially contained within a corpus of a human or an animal, especially to be contained within the corpus and extending beyond a skin of the corpus, and further comprising a compressible and / or bendable system-portion 30 to be located outside of the corpus.

In the present example, the reinforcement-means 28 is located within a wall-structure 31 of the body 24 of the pump-outflow-cannula 23 and the reinforcement-means 28 is a wire 28, in particular a wire 28 made of plastic, and / or made of a composite material, and / or made of a metal, especially made of a stainless steel.

The pump-outflow-cannula 23 includes at the proximal-end 27 a securing-means 34 for securing the proximal-end 27 to the heart 2 and / or to the associated vessel, and includes in the example of Fig. 4 a vascular graft 34 and may comprise an embedded winding for securing the vascular graft.

The design shown in Fig. 4 incorporates a standard vascular graft 34 (i.e.: diameter 8 mm) and suturing technique to the vessel wall such to create a side opening in the vessel for blood passage. The proximal-end 27 of the pump-outflow-cannula 23 is configured such to enable positioning the cannula proximal-end 27 into the body of the graft 34 through the lumen. A suture is then wrapped about the graft 34 to secure the pump-outflow-cannula 23 within it and provide a leak tight joint.

The proximal-end 27 of the pump-outflow-cannula 23 may be placed within the lumen of a vascular graft 34 (8-mm graft) attached to the aortic artery. The graft 34 is intended to prevent accidental decannulation and to reduce pressure necrosis of the vessel wall by providing a "soft" interface, and a smooth cannula to vessel transition. Placement of the pump-outflow-cannula 23 within the graft 34 and not extending the pump-outflow-cannula 23 into the aorta reduces dislodge of embolic material from the return blood flow stream.

The proximal lumen of the pump-outflow-cannula 23 is a through lumen. The periphery about the tip should be 0.315" ranging from 0.100" to 0.600".

As an alternative, the graft 34 may be sewn to the vessel outer wall. In this case, the pump-outflow-cannula 23 is positioned within the graft-lumen into the vessel. The proximal cannula tip may be bent to direct blood flow away from an inner vessel wall and the pump-outflow-cannula 23 should not be positioned exactly axial. Preferably, the graft 34 is tied-off about the cannula to inhibit blood loss through the wound.

In a further embodiment, the graft 34 is sewn to the vessel outer wall, too, and the pump-outflow-cannula 23 is positioned within the graft lumen, but not into the vessel. The graft is tied-off about the pump-outflow-cannula 23 to inhibit blood loss through the wound.

In another embodiment, no graft 34 is utilized and the pump-outflow-cannula 23 is placed through the vessel wall and secured to the vessel. The proximal cannula tip may be bent to direct blood flow away from inner vessel wall and the cannula should not be positioned exactly axial.

Regarding a further special embodiment, the graft 34 is pre-attached to the pump-outlflow cannula body. The graft 34 / pump-outflow-cannula 23 is sewn to the outer vessel wall. What is important, in this case, the cannula never enters the vessel.

A barbed fitting joint to attach the pump-inflow-cannula 1 and / or the pump-outflow-cannula 23 for example to the external blood handling system or to an other device may be provided.

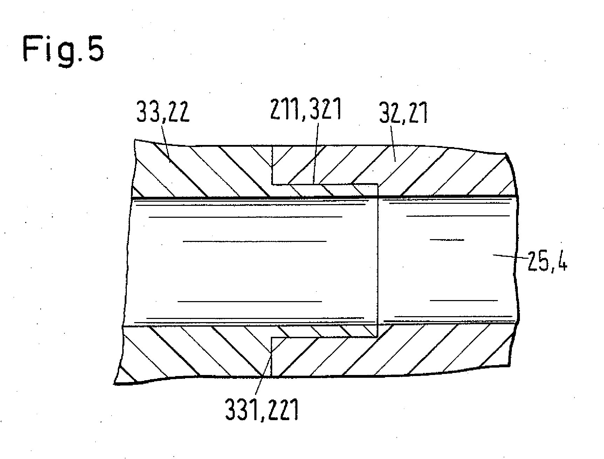

Fig. 5. displays an outflow-fitting 32 being located at the distal-end 26 of the outflow-lumen 25 of a pump-outflow-cannula 23. The outflow-fitting 32 is in the present example is a barbed 3/8" fitting 32 capable of receiving a 3/8" tube-connector 33. The outflow-fitting 32 comprises a countersink 321 to receive a fitting-wall 331 of the tube-connector 33 for attachment of the pump-outflow-cannula 23 to the external blood handling system.

The outflow-fitting 32 of the pump-outflow-cannula 23, which, of course, can also be provided as an inflow fitting 21 at the pump-inflow-cannula 1, is designed to provide a smooth transition along the inner surface to minimize or eliminate any gap that could result in thrombosis. Thrombus will form at gaps or irregular surfaces and may grow and or be accidentally released back into the blood stream. Such debris may become lodged downstream and could result in patient complications. To minimize this, the cannula body has a countersink 321, 211 to receive the barbed fitting wall and result in no irregular surface transition. This feature is, as already mentioned, common also to the pump-inflow-cannula 1.

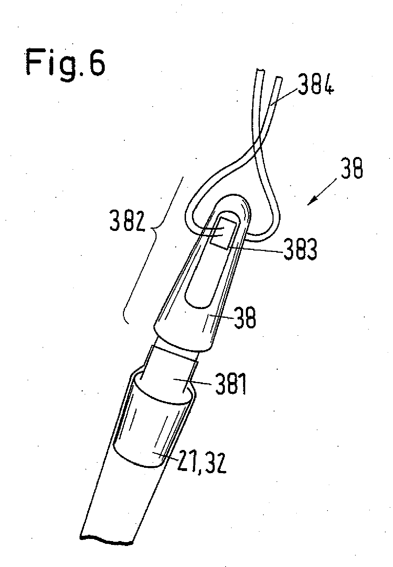

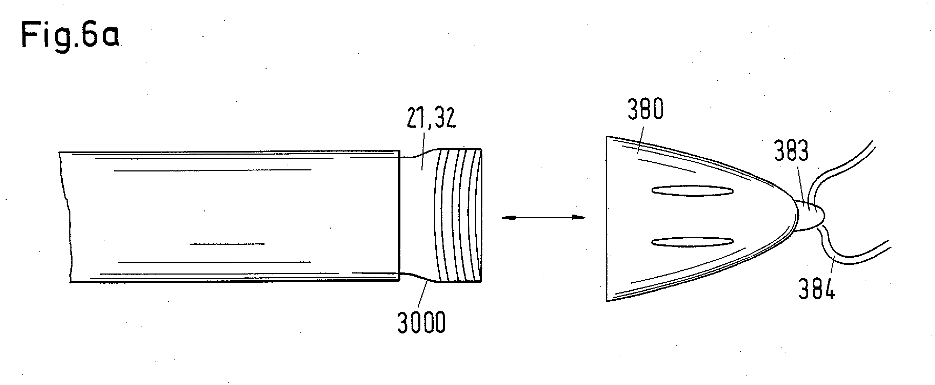

A barbed fitting 21, 32 pre-attached to the cannula should be protected during tissue tunnelling or passage from damage and contamination. Thus, a tunneler-plug 38 as shown in Fig. 6 or a tunneler-cap 380 as shown in Fig. 6a may be added to the device.

To assist the pump-inflow-cannula 1 and / or an pump-outflow-cannula 23 in tunneling through a tissue of a corpus of a patient, the tunneler-plug 38 or the tunneler-cap 380 is attached to the distal-end 6, 26 into the exit lumen of the pump-inflow-cannula 1 and / or of the pump-outflow-cannula 23, respectively. The tunneler-plug 38 contains a barbed fitting 381 on one end and a smooth tapering section 382 on the other end to ease passage as it (with its assembled cannula) dilates a lumen during passage.

Using a tunneler-cap 380 according to Fig. 6a, at the distal end of the cannulla a barbed cap-fitting 3000 is provided to attach the cannula-cap 380 to the cannula via the barbed cap-fitting 3000.

The tunneler-plug 38 and / or the tunneler-cap 380 is made of a rigid polymer or a metal (polycarbonate is preferred) and may be produced by injection molding, machining, casting or stereolithography (rapid prototyping) for short term body contact.

The tunneler-plug 38 as well as the tunneler-cap according to Fig. 6a also contains a means 383 to secure a suture 384 or an umbilical tape 384 to create a tether for assisting in pulling the assembly through the tissue.

Upon completion of passage of the cannula 1, 23 outside the body, that is outside of the corpus of the patient, the tunneler-plug 38 may be disconnected or cut-off from the cannula 1,23.

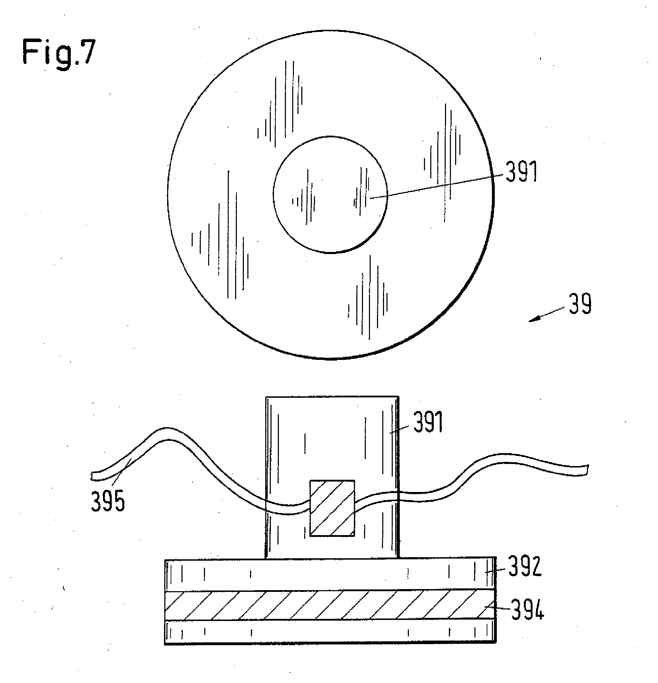

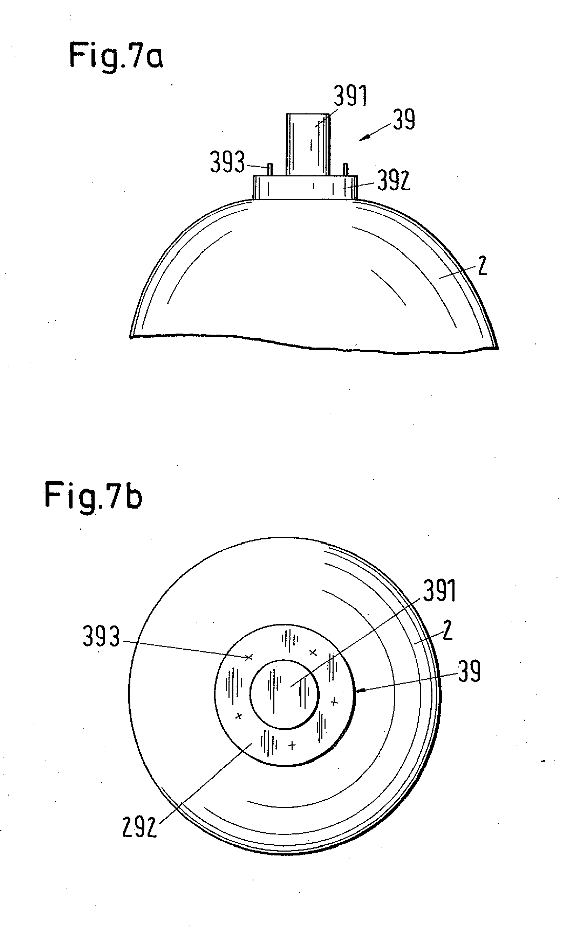

In Fig. 7 a sewing-ring accessory 39 is displayed, providing a means securely connect a cannula 1, 23, in particular a pump-inflow-cannula 1 to the heart 2 of a patient.

The sewing-ring 39 is preferably attached to the apex of the Left Ventricle of the patient's heart 2 by means of direct vision with the aid of a temporary positioning handle (not shown in Fig. 7). The sewing-ring 39 is configured intended to provide a mechanical means to secure the cannula1, 23 to the heart 2 by providing radial compression to the cannula 1, 23 while mechanically fastened to the heart tissue with sutures.

The sewing-ring 39 includes a cylindrical body 391 sized to receive the cannula outside diameter with minimal clearance. The construction of the sewing-ring 39 consists of silicone material and polyester felt. Alternatively, any hemocompatible thermoplastic or thermoset elastomer may be utilized.

The design is essentially a "top-hat" configuration with the top removed. The brim 392 provides the location for securing sutures 393 to the heart as well as position of the felt disc for tissue interface. Included in this flange is a reinforcement member 394 that is intended to minimize suture pull-through (bolster) as the felt material may not provide the needed structural resistance.

The upper "top-hat" 391 portion includes pre-assembled umbilical tape 395.

This enables less manipulation by the User to satisfactorily tie-off the cannula. The umbilical tape 395 provides a larger surface area while wrapping to resist locally cutting through the sewing-ring body and cannula wall.

Prior to implantation of e.g. the pump-inflow-cannula 1, the sewing-ring 39 is placed for example in proximity to the Left Ventricle and located such that the felt covered fabric side is facing the heart 2, and the central axis lumen is directly in-line with the apex of the left ventricle.

The aid of the aforementioned handle provides a feature to manipulate the sewing-ring 39 during handling and attachment. As displayed in Fig. 7a and Fig. 7b, six or more pledgeted sutures 393 placed through the heart tissue at even intervals approximately 60° circumferentially apart from each other around the periphery of the heart 2. The suture 393 is brought through the heart tissue approximately 1-2 cm away from the apex of the heart 2 and the final position of the sewing-ring 39. The sutures 393 are pulled through the heart muscle leading away from the pledget and towards the ventricular apex. The sutures 393 are brought through the tissue and externalized at the point that they enter the underside of the sewing-ring 39. The ends of each unique pledgeted string are brought through the cuff of the sewing-ring 39 and securely fastened.

Once positioned securely on the heart 2, a stab wound or punch is utilized to create access to the inner chamber of the heart 2. The cannula 1, 23 is then passed through the sewing-ring 39 into the heart 2. The cannula 1, 23 is positioned with the most distal drainage hole contained within the heart chamber.

The umbilical tape 395 that is pre-attached to the sewing-ring body is then wrapped about the cannula 1, 23 and secured with a knot to minimize cannula axial movement and blood leakage between the cannula 1, 23 and the sewing-ring cylinder. In addition, the sewing-ring felt disc should enable clotting of any wicked blood under the sewing-ring 39 to again inhibit blood leakage.

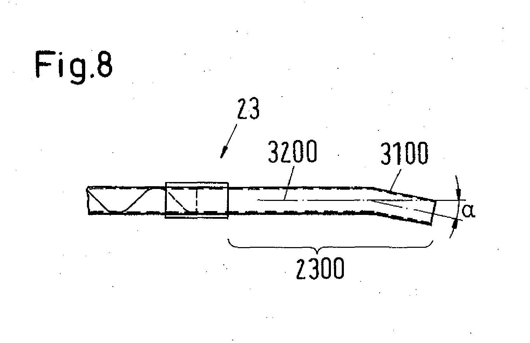

The embodiment of the proximal end of the pump-outflow-cannula 23 according to Fig. 8 incorporates no vascular graft but rather positions the cannula proximal tip directly through the vessel wall. This cannula 23 is purse-string secured to the vessel wall.

The proximal-end 2300 of this cannula 23 may be straight or bend by an angle α of 5° to 30° from an longitudinal axis 3200 to create a fluid deflection 3100 at the outlet. Such a deflection 3100 could divert blood flow from direct streaming into the opposite vessel wall and control flow direction.

The material tip should be hardened to resist deformation. This may be accomplished by solvent dipping to extract plasticisors (if PVC or vinyl) or locally dipped into a harder material durometer.

Alternative embodiments are additionally possible. Co-axial transseptal percutaneous cannula is known from the state of the art (CardiacAssist Inc., Pittsburgh, PA -TandemHeart Percutaneous Transseptal Ventricular Assist (PTVA) System) requiring a single vascular puncture. Such cannulae may be placed through a major vessel (internal jugular vein, external jugular vein, inferior vena Cava) to access the heart.

This pump Outflow cannula proximal ends reside in right atrium and the pump Inflow cannula proximal end resides in the left atrium - piercing through the septum. Angled holes at the proximal pump-outflow-tip could be utilized to reduce trauma.

The features described in this disclosure may be applied to other blood handling cannulae accessed through smaller vessels for procedures such as dialysis, active (suction or pump assisted) autotransfusion, standard cardio-pulmonary bypass surgery, etc.. Of particular consideration for these other applications is the angulated holes in the cannula and associated radii.

The cannulae may be scaled up or down depending upon the desired blood flow rate and pressure drop required across the system. As such, connector fittings and tubing sizes may be compatibly substituted.

The tapered blood flow holes may be adapter to cannulae utilized for extracorporeal or intracorporeal support Systems.

The cannula may be multi-lumen providing a means for passage of secondary devices utilized for diagnostics or therapeutic applications. A second or third lumen may be utilized for features such as temperature sensing, blood gas sensing, pressure measurements, guidewire passage, catheter passage, flushing, vascular injections, etc.

Cannulae may be treated or lined with clot resistant coatings. Additionally, the cannulae may be utilized for standard up-to-six-hour cardiopulmonary surgical support.

Some detail elements, such as the angles holes, may have other applications such as use in dialysis catheters.

It is understood that the invention is not only related to the special embodiments discussed above but also are further embodiments included. In particular, the invention relates to all advantageous combinations of the discussed embodiments.

## Claims

1. A pump-outflow-cannula providing a blood conduit from an external blood handling system to a heart (2) and / or to an associated vessel, said pump-outflow-cannula comprising a body (24) encompassing an essentially axially extending outflow-lumen (25) having a distal-end (26) for an introduction of blood from said blood management system into the outflow-lumen (25), and having a proximal-end (27) for an attachment of the outflow-lumen (25) to the heart (2) and / or to the associated vessel, **characterized in that** said body (24) of the pump-outflow-cannula comprises a reinforcement-means (28).

2. A pump-outflow-cannula in accordance with claim 1, comprising a reinforced body-portion (29) to be essentially contained within a corpus of a human or an animal, especially to be contained within the corpus and extending beyond a skin of the corpus, and comprising a compressible and / or bendable system-portion (30) to be located outside of the corpus.

3. A pump-outflow-cannula in accordance with anyone of claims 1 or 2, wherein the reinforcement-means (28) are located within a wall structure (31) of the body (24) of the pump-outflow-cannula.

4. A pump-outflow-cannula in accordance with anyone of the preceding claims, wherein the reinforcement-means (28) is a wire (28), in particular a spring (28) made of plastic, and / or made of a composite material, and / or made of a metal, especially made of a stainless steel.

5. A pump-outflow-cannula in accordance with anyone of the preceding claims, wherein at the distal-end (26) of the outflow-lumen (25) an outflow-fitting (32), in particular a barbed 3/8" fitting (32) capable of receiving a 3/8" tube-connector (33), preferably a fitting (32) comprising a countersink (321) to receive a fitting-wall (331) of the tube-connector (33), is provided for attachment of the pump-outflow-cannula to the external blood handling system and / or wherein the distal-end (26) is straight or angled according to an distal-angle, the distal-angle preferably ranging from 0° to 40° from a outflow-lumen-axis (37), preferably ranging from 1° to 30°, in particular being 10°.

6. A pump-outflow-cannula in accordance with anyone of the preceding claims, wherein the proximal-end (27) comprises a securing-means (34) for securing the proximal-end (27) to the heart (2) and / or to the associated vessel, in particular a vascular graft (34).

7. A pump-outflow-cannula in accordance with anyone of the preceding claims, wherein the proximal-end (27) of the outflow-lumen (25) is a through lumen comprising at a hardened proximal-tip (35), and the periphery about the proximal-tip (35) ranges from 0.005" to 0.800", in particular from 0.100" to 0.600", and is preferably 0.315".

8. A pump-outflow-cannula in accordance with anyone of the preceding claims, wherein a surface of the pump-outflow-cannula is biocompatible to blood and / or a tissue and / or the pump-outflow cannula is made of a polycarbonate-based urethane, especially made of carbothane and / or another urethane und / or PVC, and / or vinyl materials, and / or elastomeric materials.

9. A pump-outflow-cannula in accordance with anyone of the preceding claims, wherein a depth-indicator is provided for indicating a placement depth into the vessel.

10. A pump-outflow-cannula in accordance with anyone of the preceding claims, wherein a positionable securing-grommet (36) is provided to secure the pump-outflow-cannula.

11. A pump-outflow-cannula in accordance with anyone of the preceding claims, wherein an introducer with a hub including a guide wire is provided, in particular a guide wire having a diameter of 0.038", to permit access into a vessel, in particular into the aorta.

12. A blood managing system comprising a pump-outflow-cannula (23) in accordance with anyone of the preceding claims.

13. A blood managing system in accordance with claim 12, wherein a tunneler-plug (38) is provided to assist the pump-outflow-cannula (23) in tunnelling through a tissue of a corpus.

14. A blood managing system in accordance with anyone of claims 12 or 13, wherein a blood handling system, in particular a blood pump, or an oxygenator, especially an extracorporeal membrane oxygenator, or a dialysis apparatus, or an other medical apparatus is provided for connection with the pump-outflow-cannula (23).
